# EUROPEAN PATENT SPECIFICATION

(11) **EP 1 860 110 B1**
(45) Date of publication and mention of the grant of the patent: **05.08.2015**
(21) Application number: 07008594.9
(22) Date of filing: 27.04.2007
(51) Int. Cl.: A61K 31/715, C08B 37/16

(54) **Cyclodextrins functionalised with etodolac as specific site release agents**
Mit Etodolac als ortsspezifische Freisetzungswirkstoffe funktionalisierte Cyclodextrine
Cyclodextrines fonctionnalisées avec etodolac en tant qu'agents de libération de sites spécifiques

(30) Priority: 05.05.2006 IT MI20060894
(43) Date of publication of application: 28.11.2007
(73) Proprietor: Universita' Degli Studi Di Catania, 95124 Catania (IT)
(72) Inventor: Rizzarelli, Enrico, 95124 Catania (IT); Vecchio, Graziella, 95124 Catania (IT); Puglisi, Antonino, 95124 Catania (IT); La Mendola, Diego, 95124 Catania (IT)
(74) Representative: Minoja, Fabrizio

(56) References cited:
- WANG N ET AL: "Regioselective synthesis of cyclodextrin mono-substituted conjugates of non-steroidal anti-inflammatory drugs at C-2 secondary hydroxyl by protease in non-aqueous media" BIOORGANIC & MEDICINAL CHEMISTRY, ELSEVIER SCIENCE LTD, GB, vol. 13, no. 11, 1 June 2005 (2005-06-01), pages 3667-3671, XP004873527 ISSN: 0968-0896
- UEKAMA K ET AL: "CYCLODEXTRIN DRUG CARRIER SYSTEMS" CHEMICAL REVIEWS, ACS,WASHINGTON, DC, US, vol. 98, no. 5, July 1998 (1998-07), pages 2045-2076, XP000771829 ISSN: 0009-2665
- MILIC-ASKRABIC, J. ET AL: "Etodolac and solid dispersion with .beta.- cyclodextrin" DRUG DEVELOPMENT AND INDUSTRIAL PHARMACY , 23(11), 1123-1129 CODEN: DDIPD8; ISSN: 0363-9045, 1997, XP009086207

## Description

The present invention relates to conjugates of cyclodextrin with anti-inflammatory products and the use thereof as site-specific medicaments.

More particularly, the invention relates to conjugates of cyclodextrin with anti-inflammatory compounds having the formula (I):

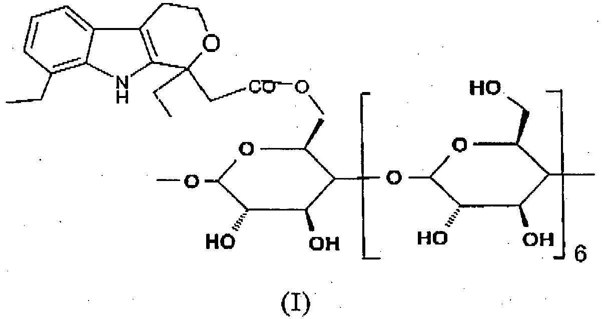

Compounds of formula (I) were obtained functionalising a cyclodextrin, particularly β-cyclodextrin, at the 6-position with (+)etodolac. Conjugates with S(+)-etodolac and R(-)etodolac were obtained. The compounds of the invention have anti-inflammatory activity and can be selectively transported to the intestine thanks to the functionalisation with cyclodextrin.

### TECHNICAL BACKGROUND

Cyclodextrins (CDs) are widely used in the pharmaceutical industry as drug-complexing agents, stabilizers for unstable drugs and carrier systems for the controlled release. In addition, CDs are used to reduce or prevent gastro intestinal or ocular irritations, to reduce or eliminate unpleasant tastes or smells, to prevent drug-to-drug interactions or formation of amorphous powders or microcrystals.

In general, cyclodextrins act as carriers towards biological membranes (e.g. skin, mucous membranes, cornea) and being hydrophilic molecules they remain outside the membrane to which the complexed drug is released. More specifically, the release rate can be modulated to obtain an immediate, delayed or prolonged release for certain drugs that use these macrocycles as delivery systems (F. Hirayama, K. Uekama Adv. Drug Deliv. Rev. (1999) 36, 125).

An immediate release is usually related to an increase in the solubility of the drug induced by the presence of CDs, which allows to increase the concentration of the drug in solution, and therefore the absorption. This is particularly useful in the case of anti-pyretics, vasodilators, analgesics etc. which are used in emergency situations. For certain systems, a slow (prolonged) release is observed which allows the number of administrations of the drug to be reduced while maintaining adequate blood concentrations. Recently two strategies to increase the potentiality of cyclodextrins as delivery agents have been described in the literature: a) the synthesis of covalent derivatives with the pharmaceutically active molecule with the aim of guaranteeing the release of the drug in the areas where absorption takes place; b) the functionalisation of CD with messenger molecules such as opioid peptides or oligosaccharides which act as specific binders for certain membrane receptors. The cavity of these derivatives can transport the drug ensuring the specific site-release.

The direct functionalisation with drugs seems to be a promising strategy in order to i) increase stability, mainly to proteases in the case of a drug of a peptide nature, ii) improve solubility and iii) ensure the site-specific release. The drug covalently bonded to CD is a novel molecule and therefore can lose or modify its pharmacological activity. Therefore, the synthesized derivate should guarantee the release of the drug in the area where absorption takes place. Various drugs and receptors such as doxorubicin, methycillin, biological peptides such as enkephalin, neuropeotide P, gastrin, a cysteine protease inhibitor (Ac-Leu-Leu-Nle-H), carnosine, have been linked to CDs,

Peptides have usually been bonded through condensation of a peptide carboxylic group with 6-amino-6-deoxy-β-CD either directly or through spacers. The enkephalin derivative is apparently less efficient than enkephalin in the substrate binding assay. Another interesting derivative is that obtained functionalising CD with the cysteine protease inhibitor, which maintains inhibition ability analogous to that of the unbdonded inhibitor.

A further advantage of functionalisation is that site-specific transport of the drug is obtained, specially to the intestine (F. Hirayama, K. Uekama Adv. Drug Deliv. Rev. (1999) 36, 125). The simple inclusion in fact does not usually allows to efficiently attain this objective. An example are drugs which are absorbed in the colon: a simple inclusion complex would release the drug in the stomach, while a functionalised derivative is able to move through the gastric tract and be degraded in the colon into small oligosaccharides by the enzymes produced by the intestinal microflora.

One of the first compounds proposed as a controlled release system in the colon is β-CD functionalised with diphenylacetic acid (K. Uekama, K. Minami, F. Hirayama J. Med. Chem. (1997) 40, 2755) through esterification of a 6-hydroxyl of β-CD. Derivates of diphenylacetic acid α, β, γ-CD functionalised through amidic or ester bonds have subsequently been synthesized. The solubility of the conjugates depends on the CD and the conjugate with β-CD shows low solubility, while those with the other CDs have remarkably greater solubility than the acid itself. Compared with diphenylacetic acid ethyl ester, which is easily hydrolyzed in the liver and gastrointestinal tract, the esters deriving from α- and γ-CD show a greater stability to hydrolysis and release the drug only in the colon and caecum. In case conjugation is obtained through an amido bond, the release does not take place, but the drug remains linked to di- and tri- saccharides that are not further degraded due to the presence of the amido bond. More recently, CD conjugates with prednisolone have been synthesized, specific for inflammatory pathologies of the intestinal tract (H. Yano, et al., J. Pharm. Sci. (2001) 90, 493; F. Hirayama, et al. J. Control. Rel. (2002) 79, 103). Prednisolone as such, when orally administered, is absorbed in remarkable quantities in the stomach, thus causing serious systemic side effects that affect the therapeutic activity. Conjugates have been prepared by ester or amido bond of succinic acid-derivatized prednisolone to CD. The derivatives through the ester bond seem to be promising for the purposes of a slow release in the colon, being able to significantly prevent the absorption in the stomach with its connected side effects. A further advantage of prednisolone CD functionalisation is the increase in the solubility of the conjugate compared with the drug itself.

Non steroid anti-inflammatory drugs (NSAIDs) are a class of drugs widely used as anti-pyretics, pain killers, anti-arthritics and, recently, as agents able to protect against Alzheimer's disease (AD). It is hypothesized that the action mechanism of anti-inflammatory drugs is mainly based on the inhibition of cyclooxygenase (COX), also known as prostaglandin synthetase, a key enzyme in the metabolization of arachidonic acid to prostaglandin. Two isoforms of COX have been characterized, COX-1, a constitutive form that has cytoprotective activity in the gastrointestinal tract and the kidneys, and COX-2, a mitogen inducible form, that mediates inflammatory processes. Many NSAIDs non-selectively inhibit both COXs; the side effects on the digestive apparatus are mainly related to COX-1 inhibition. The damage to the gastric apparatus results in fact both from the reduced production of protective prostaglandins, essential to maintain the integrity of the gastrointestinal tract and homeostasis and, to a lesser extent, from the acidic properties related to the presence of the free carboxylic group, present in most NSAIDs (topical effect). The incidence of these side effects can exceed 30% and can even involve the need to abandon the therapy. The pharmacological activity is essentially correlated to the inhibition of COX-2 induced by inflammation and, with the aim of reducing side effects, the new generation NSAIDs selectively inhibit COX-2. Also traditional drugs such as etodolac, meloxicam and nimesulide proved preferential inhibitors of COX-2 (A. Bennett et al., Exp. Opin. Pharmacother. (2001) 2, 1859).

In addition to the traditional therapeutic indications of anti-inflammatory drugs, today new applications of NSAIDs are being studied in the cure and prevention of Alzheimer's disease (AD) (P.S.Aisen, MD J. Pain Symptom (2002) 23, S35), adenomas and colon tumors (D.K. Rex, MD, FACG J. Pain Symptom (2002) 23, S41). To date, no effective therapies for the cure of AD primary symptoms exist. It has been demonstrated that, beside the neurodegeneration process characteristic of the pathology, an inflammatory process occurs and, in the light of that, the role of anti-inflammatory drugs has recently been evaluated both in the prevention and the cure of the disease. Recent studies suggest that COX-2 selective inhibitors are more advantageous than non-selective NSAIDs. COX-2 has in fact a central role in the neurodegenerative process, it is overexpressed in the case of excitotoxic lesions and its expression in the brain is correlated to the pathological formation of amyloid plaques. The inhibition of COX-2 is therefore proposed as a therapeutic approach for Alzheimer's disease.

The role of anti-inflammatory drugs also seems to be recognised in the case of other diseases, such as tumours of the colon. It was recently found that NSAIDs can be successfully used to prevent colorectal tumours. Their action is believed to be dependent on COX inhibition. COX-2 is overexpressed in the gastrointestinal mucosa of patients with adenomas or colorectal tumours, but is substantially undetectable in normal patients. It has been demonstrated in vivo that inhibition of COX-2 inhibits angiogenesis and promotes apoptosis (programmed cell death) in colorectal cancer. It has also been proved that the risk of development of colorectal tumours is considerably reduced by the use of anti-inflammatories, to a dose-dependent extent. In view of the overexpression of COX-2, anti-inflammatories which are selective COX-2 inhibitors have been studied, with good results in patients with disorders that predispose them to colorectal tumours, as regards both the development of the disease and reduction of colorectal polyposis. Their role as selective COX-2 inhibitors also seems to be significant in the case of some tumour lines in prostate cancer (T. Samijo et al., Inter. J. Urol. (2001) 8, S35): the proliferation rate is strongly inhibited by anti-inflammatories, which induce apoptosis of the tumour cells. The proliferation rate of normal cells remains unchanged.

In view of these findings, research into anti-inflammatories is a particularly interesting field, as regards both the synthesis of new molecules and methods of delivering traditional molecules. Numerous inclusion complexes of cyclodextrins with NSAIDs (ibuprofen, ketoprofen, indometacin, diclofenac, naproxen, nimesulide, etodolac etc.) have therefore been described, characterised and studied in pharmacological terms, and some of them are available on the market. The presence of cyclodextrin is designed to reduce gastrointestinal damage, and improve the release and dissolution rate of the drug. However, the results are rather modest in terms of both solubility and bioavailability. Inclusion is insufficient (i) to eliminate the irritant effect of contact with the gastric mucosa due to the carboxyl group present in the anti-inflammatory drug, because of the presence of a considerable amount of free drug, and (ii) to make the drug completely soluble, in order to facilitate its administration and reduce the effect on the gastric apparatus. Under these circumstances, the study of simple methods of delivering NSAIDs is particularly interesting. In particular, anti-inflammatories can be delivered in such a way as to be absorbed into the colon, thus ensuring: 1) protection against the topical effect due to the carboxyl group, which is also present in selective COX-2 inhibitors; 2) delivery of the drug to the required site, which could boost its activity, especially in the case of colon tumours or polyposis. Functionalisation of cyclodextrins with the drugs in question would guarantee these benefits.

The drug would be released unmodified, and the conjugate would therefore maintain the same therapeutic indications and the same pharmacological characteristics as the free drug.

Cyclodextrins as chiral entities allow the separation of optical isomers, which is important in the field of chiral NSAIDs. For some anti-inflammatories, such as etodolac, only racemic mixtures are commercially available, although it has been demonstrated that the two enantiomers possess different activities. S-etodolac possesses anti-inflammatory activity (L.G. Humber et al., J. Med. Chem. 1986, 29, 871), whereas R-etodolac is inactive as an anti-inflammatory but exhibits anticancer activity (WO 01/06990). The distribution *in vivo* of the two enantiomers is highly stereoselective, with a high concentration of the R enantiomer and a much lower concentration of the S form in the plasma. In this respect the behaviour of etodolac is untypical of the other NSAIDs, for which the concentrations of the two enantiomers in the plasma remain comparable, although the S isomer is the active one. The behaviour of etodolac provides an example of the usefulness of optically pure molecules.

Conjugates of cyclodextrins with known anti-inflammatories are already being studied for diseases such as tumours of the colon (W-S. Chen et al., Intern. J. Cancer (2001) 91, 894), and *in vitro* on the cells of prostate tumours (T. Samijo et al., Inter- J. Urol- (2001) 8, S35), leukaemia and myelosis (WO 01/06990). A specific example of etodolac bound to cyclodestrin is disclosed by wang et al. (Bioorg. Med. Chem. 13 (2005) p 3667-71). myelosis (WO 01/06990).

### DISCLOSURE OF THE INVENTION

The invention relates to conjugates of cyclodextrin with etodolac (1,8-diethyl-1,3,4,9-tetraydropiran[3,4-b]indole-1-acetic acid).

Functionalisation provides a specific site-release in the intestine, analogous to that reported in literature for similar systems (H.Yano et al., J.Pharm.Sci. (2001) 90, 493; F.Hirayama et al., J.Pharm.Sci. (2001) 90, 2103; F.Hirayama et al., J.Control. Rel. (2002) 79, 103; K.Uekama et al., J.Med.Chem. (1997) 40, 2755). This approach seems particularly interesting in the case of intestinal pathologies. Numerous patents in fact demonstrate the interest towards the local release of drugs in the intestine, specially in relation to intestinal polyposis. The possibility to use β-cyclodextrin etodolac conjugates allows to suitably target the drug.

The conjugates of the invention can be prepared by reacting a cyclodextrin derivative at the 6- position hydroxyl, for instance a cyclodextrin 6-tosylate, with an etodolac salt. The use of β-cyclodextrin is preferred, although also other known cyclodextrins or derivatives thereof can be used.

In the β-cyclodextrin etodolac conjugates of the invention, functionalization allows to isolate the two (S)- and (R)- diastereomers. As the two enantiomers are known to have different pharmacological activities, particularly S-etodolac being the best COX inhibitor (WO 95/27713), the use of optically pure etodolac conjugates is advantageous in terms of activity/side effects ratio.

A further advantage according to the invention is the solubility of the mixture of the diastereomers of the conjugates, markedly more water-soluble than etodolac itself. The two isolated diastereomers show different solubilities in water, higher in the case of the R-etodolac conjugate (6.8 g/l).

The conjugates of the invention are useful for the preparation of medicaments for the colon-selective release.

The compositions of the invention will be mainly administered through the oral route, in the form of capsules, tablets, protracted-release or enteric-coated forms, prepared using conventional techniques and excipients. Dosages will be of the same order as those known for etodolac or even lower, in view of the improved characteristics of the conjugates of the invention.

The invention will be illustrated in further detail in the following Example.

### Synthesis: Experimental Part

β-Cyclodextrin (CD) (Sigma) was dried under vacuum (10⁻² mm Hg) for about 24h at 80°C, using a P₂O₅ trap. Etodolac was used as the sodium salt. Thin layer chromatography (TLC) was carried out on silica gel plates (60F-254, 0.25 mm, Merck) and the products, not visible under UV light, were revealed using a 5% anysaldehyde solution in ethanol (containing 5% H₂SO₄). HPLC was carried out on an Econosphere analitycal (5 µ) and preparative (10 µ) column (Alltech). NMR spectra were recorded on a Varian UNITY PLUS 500 MHz spectrometer. DSS was used as an external standard. ¹H-NMR spectra were assigned by COSY, TOCSY, ROESY experimental.

### EXAMPLE

### Synthesis of 6-O-[1,8-diethyl-1,3,4,9-tetrahydropyrano(3,4-b)indole-1-acetyl]-β-cyclodextrin

6-O-tosyl-β-cyclodextrin (12 g) (R.P. Bonomo et al., Inorg. Chem., 1991, 30, 2708) was added to an etodolac solution in 100 ml of DMF (in stoichiometric amounts). The reaction was carried out at 100°C under stirring. After 30h the solvent was evaporated off under vacuo. The reaction mixture was purified by Rp8 column chromatography, using water-MeOH as the eluent. The product was eluted at a MeOH concentration of 70%. TLC: Propanol-AcOEt-H₂O-NH₃ 5:2:3:3 Rf= 0.61. The recovered product consists of two diastereomers: the conjugate with S(+)-etodolac and that with R(-) etodolac. Yield in diastereomer mixture: 35%.

The two diastereomers were separated by preparative HPLC with Econosphere ODS column (Alltech, 22x250 mm), eluent H₂O-MeOH 40 → 70 (40', flow rate 10 ml/mm). The conjugate with S-etodolac eluted first. The configuration of cyclodextrin-conjugated etodolac was assigned by hydrolysing one of the diastereomeric products, namely the one eluted first from the preparative HPLC column and then measuring the optical rotary power of the etodolac released. This product was hydrolyzed by suspending it in 1% NaOH aqueous solution for 2h at room temperature. After completion of the reaction, the mixture was acidified and etodolac was extracted by CH₂Cl₂, dried, dissolved in EtOH and the measured [α]_{D}²⁰ optical rotary power = 31.2 (c=0.2, ethanol) indicated that the product was etodolac S(+)-enantiomer.

The two diastereomers were characterized singularly.

*6-O-[1(S)1,8-diethyl-1,3,4,9-tetrahydropyrano(3,4-b)indole-1-acetyl]-β-cyclodextrin:* ES-MS 1404 *m*/*e;* ¹H NMR (CD₃OD) in ppm 0.76 (3H, t, CH₃ of the ethylene chain bound to tetrahydropyran), 1.35 (3H, t, CH₃ of the ethylene chain of the benzene residue), 2.07 (2H, m, CH₂ of the ethylene chain bound to the tetrahydropyran ring), 2.76 (2H, m, H-3 of the tetrahydropyran); 2.89 (3H, m, CH₂ of ethylene chain bound to the benzene and a CH₂ proton in α to the carboxyl group); 3.16 (1H, d, other CH₂ proton in α to carboxyl group); 321 (1H, dd, 2A); 3.25 (1H, t, 4-A); 3.28-3.53 (12H, m, 2-H and 4-H of CD); 3.54-3.99 (26H, m, 5-,6,-3-H of CD); 4.03 (2H, m, H-2 of tetrahydropyran); 4.77 (d, 1A, with HOD peak), 4.83 (1H, d, 1G); 4.98 (5H, d, other H-1 of CD); 6.91 (1H,d, proton in ortho to the ethylene chain of the benzene ring), 6.96 (1H, t, proton in metha to the ethylene chain); 7.27 (1H, d, proton in para to the ethylene chain). Circular dichroism: 205 nm Δε = 3; 233 nm Δε = -16.

### 6-O-[1(R)1,8-diethyl-1,3,4,9-tetrahydropyran(3,4-b)indole-1-acetyl]-β-cyclodextrin

ES-MS 1404 m/e; ¹H NMR (D₂O): in ppm 0.65 (3H, t, CH₃ of ethylene chain bound to tetrahydropyran), 1.25 (3H, t, CH₃ of ethylene chain of benzene), 1.91 (1H, m, H of CH₂ bound to tetrahydropyran), 2.03 (1H, m, H of CH₂ bound to tetrahydropyran); 2.69 (2H, m, H-3 of tetrahydropyran); 2.72 (2H, m, CH₂ of ethylene chain of benzene); 2.98 (2H, s, CH₂ in a to the carboxyl group); 3.15 (1H, t, 4-A); 3.20-4.00 (33H, m, 5-,6,-3-,2-,4-H of CD and H-2 of tetrahydropyran); 4.60 (1H, d, 6A); 4.75 (1H, d, H-1), 4.83 (2H, m, H-1 and 1A); 4.99 (1H, d, H-1 of CD); 4.97 (3H, m, H-1); 6.96 (1H,d, proton in ortho to the ethylene chain of benzene), 7.03 (1H, t, proton in metha to the ethylene chain); 7.30 (1H, d, proton in para to the ethylene chain). Circular Dichroism: 205 nm Δε = -4; 233 nm Δε = 16.

## Claims

1. A compound of formula (I): 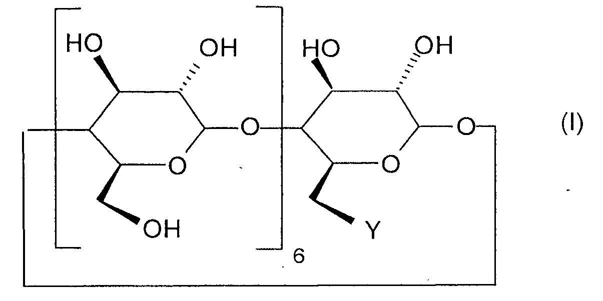 wherein Y is the acyl residue of R- 1,8-diethyl-1,3,4,9.tetrahydropyran(3,4-b)indole-1-acetic acid.

2. Pharmaceutical compositions containing the compound of claim 1 in admixture with suitable carriers.

3. The use of the compound of claim 1 for the preparation of medicaments with colon site-specific delivery.

## Patentansprüche

1. Verbindung der Formel (I): 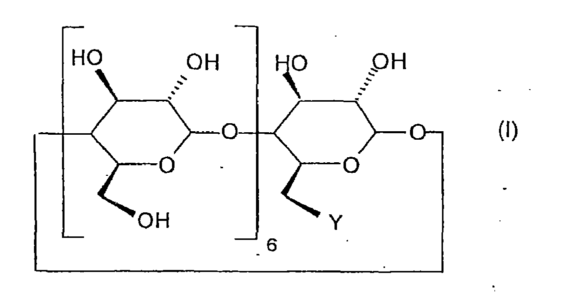 worin Y der Acylrest von R-1,8-Diethyl-1,3,4,9-tetrahydro-pyran(3,4-b)indol-1-essigsäure ist.

2. Pharmazeutische Zusammensetzungen enthaltend die Verbindung nach Anspruch 1 in einem Gemisch mit geeigneten Trägerstoffen.

3. Verwendung der Verbindung nach Anspruch 1 zur Herstellung von Medikamenten mit Colon-spezifischer Abgabe.

## Revendications

1. Composé de formule (I) : 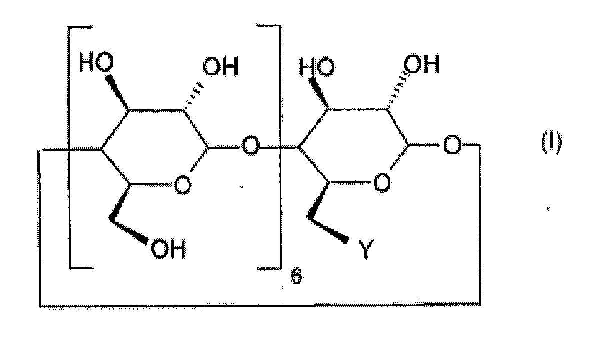 dans lequel Y est le résidu acyle de l'acide R-1,8-diéthyl-1,3,4,9-tétrahydropyran(3,4-b)indole-1-acétique.

2. Compositions pharmaceutiques contenant le composé selon la revendication 1 en mélange avec des vecteurs appropriés.

3. Utilisation d'un composé selon la revendication 1 pour la préparation de médicaments à libération spécifique au niveau du côlon.
